# EUROPEAN PATENT APPLICATION

(11) **EP 2 784 708 A1**
(43) Date of publication of application: **01.10.2014**
(21) Application number: 14161724.1
(22) Date of filing: 26.03.2014
(51) Int. Cl.: G06F 19/00

(54) **Method and system for patient flow**

(30) Priority: 28.03.2013 US 201361806159 P
(71) Applicant: Medworxx Inc., Toronto, Ontario M5H 2K1 (CA)
(72) Inventor: Vanier, Larry, London, Ontario N6K 2W7 (CA); Matlow, Daniel, Thornhill, Ontario L4J 6N9 (CA)
(74) Representative: Kindermann, Peter

(57) **Abstract**

A system for integrating multiple databases, the system having at least one server, the at least one server maintaining a first database and a second database; a plurality of terminals, each of the plurality of terminals being configured to: request data from the at least one server; receive the requested data; correlate data from the first database with data from the second database; and display data from the first database with correlated data from the second database.

## Description

### FIELD OF THE DISCLOSURE

The present disclosure relates to databases, and specifically to integrated database systems used in a hospital setting.

### BACKGROUND

For a hospital to function smoothly and effectively, relevant information relating to the care of a patient should be available to any caretaker which requires that information, and that this information be up-to-date and accurate. Moreover, many administrative decisions, such as resource allocations are best taken with complete knowledge of all patient needs.

However, hospitals today rely on disjoint programs with multiple databases, each having different functions and serving different clients. These programs/databases are typically incompatible with each other, which results in multiple independent systems instead of one unified system. This results in sub-optimal planning, inefficiencies, redundancies, and even medical errors.

### SUMMARY

The present disclosure provides a system for integrating multiple databases, comprising at least one server, the at least one server maintaining a first database and a second database; a plurality of terminals, each of the plurality of terminals being configured to: request data from the at least one server; receive the requested data; correlate data from the first database with data from the second database; display data from the first database with correlated data from the second database.

The present disclosure further provides a method for integrating multiple databases, comprising requesting data from at least one server, the at least one server maintaining a first database and a second database; receiving the requested data; correlating data from the first database with data from the second database; displaying data from the first database with correlated data from the second database

### BRIEF DESCRIPTION OF THE DRAWINGS

The present application will be better understood with reference to the drawings, in which:
**Figure 1** is a conceptual diagram of an exemplary system in accordance with at least one embodiment of the present disclosure.
**Figure 2** is a conceptual diagram of an exemplary system in a hospital setting in accordance with at least one embodiment of the present disclosure.
**Figure 3** is a block diagram of a terminal according to at least one embodiment of the present disclosure.
**Figure 4** is an exemplary user interface according to at least one embodiment of the present disclosure.
**Figure 5** is a block diagram of a method for correlating data according to the present disclosure.
**Figure 6** is a graphical representation of various databases according to at least one embodiment of the present disclosure.
**Figure 7** is an exemplary user interface according to at least one embodiment of the present disclosure.
**Figure 8A** is an exemplary report produced by at least one embodiment of the present disclosure.
**Figure 8B** is an exemplary report produced by at least one embodiment of the present disclosure.
**Figure 8C** is an exemplary report produced by at least one embodiment of the present disclosure.
**Figure 8D** is an exemplary report produced by at least one embodiment of the present disclosure.
**Figure 8E** is an exemplary report produced by at least one embodiment of the present disclosure.
**Figure 8F** is an exemplary report produced by at least one embodiment of the present disclosure.

### DETAILED DESCRIPTION OF THE DRAWINGS

In accordance with the embodiments described herein, an integrated system which keeps track of all relevant information, both clinical, logistical and operational, and which provides each decision-maker with easily accessible and relevant information is provided. In particular, the present disclosure describes a system which provides a comprehensive real-time understanding of appropriateness of the care setting according to the patient's care intensity needs; care plan delays and resource needs by location; and presents through intuitive graphical and numerical representations current demands and capacity for resources plus trends and projections.

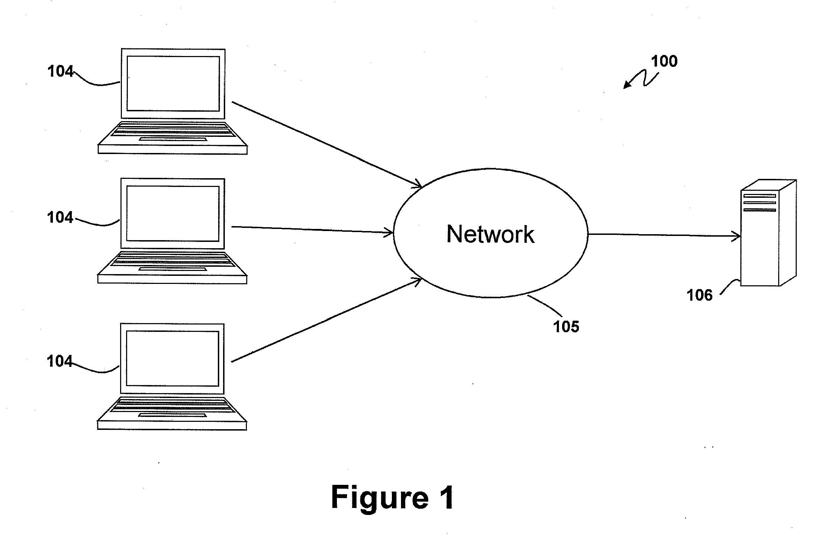

Reference is made to **Figure 1**, which shows an exemplary system **100** in accordance with at least one embodiment of the present disclosure. As seen in **Figure 1**, a plurality of terminals **104** are connected to server **106** via network **105**. The present disclosure is not limited to any particular kind of network, nor terminal. For example, the network may be a wireless network, such as for example a Wi-Fi network, or may be a wired network, such as for example an Ethernet network, a combination of the two, or may be any other type of networks known in the art.

Similarly, the terminals **104** may be a desktop computer, a laptop, a tablet, a mobile device, or other computing devices capable of being connected to network **105**. In various embodiments, terminals 104 may display reports and provide forms, screens, dashboards and the like for allowing a user of system **100** to interface with the system.

Server **106** may be a single server, or a plurality of servers that communicate with each other.

Server **106** maintains a plurality of databases. Terminals **104** may send queries to server **106** for data from the databases. The queries are carried over network **105** and the results of the queries are returned over network **105** to the terminals **104**.

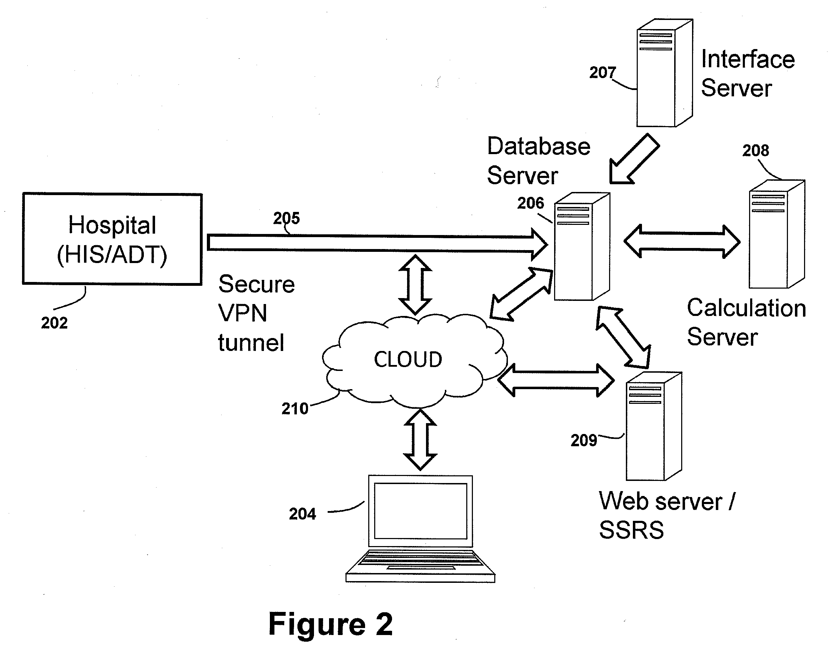

In at least one embodiment, the architecture of **Figure 1** is integrated into a hospital setting, as illustrated in **Figure 2**. However, the architecture of **Figure 1** may be adapted as would be appreciated by those skilled in the art to different settings, and the use of a hospital setting is merely exemplary.

In the embodiment of **Figure 2**, the system may communicate and share data with other systems, such as the Hospital Information System (HIS) and Admit/Discharge/Transfer (ADT), as shown at block **202**.

In the embodiment of **Figure 2**, data is communicated between various elements using a secure connection **205**. Such secure connection may, for example, utilize a secure socket layer (SSL) protocols and may form a virtual private network (VPN) in some embodiments. In other embodiments, different security as known in the art may be used for connection **205.** In at least one embodiment, data is communicated over the network according to the Health Level 7 (HL7) protocol. Cloud storage may be provided at **210.**

As seen in **Figure 2****,** a plurality of servers maintain databases and provide services to the various terminals. Specifically, **Figure 2** shows a database server **206,** for holding all clinical and operational data, an interface server **207,** a calculation server **208** and a web server **209,** which may include Structured Query Language (SQL) Server Reporting Services (SSRS). However, this configuration is provided for illustrative purposes only and is not intended to be limiting.

The various servers interact as described below. The Interface Server receives data messages from source systems, parses the message into information fields and populates them into the databases. The Interface Server also sends derived information to destination systems. The Calculation Server processes data in real-time to pre-calculate key information for consistent and faster presentation on screens and reports. The Web Server / SSRS processes information and provides the presentation layer of the system for user input, information sharing displays and reports.

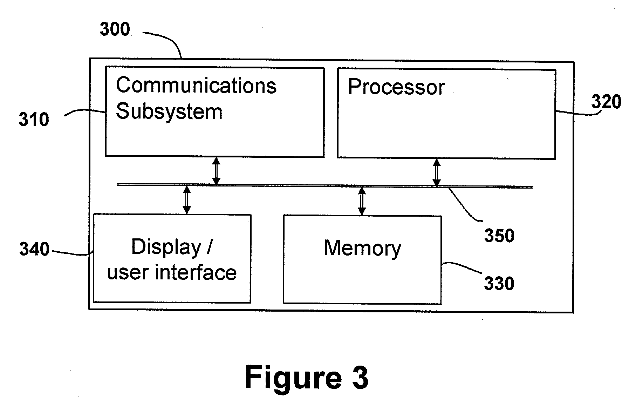

Further, a simplified block diagram of an exemplary terminal is provided with reference to **Figure 3**. As seen in **Figure 3**, the terminal **300** includes a communication subsystem **310**. The communication subsystem may allow for wireless or wired communication, and enables the terminal to communicate with the servers of the present disclosure.

Terminal **300** further includes a processor **320**, which is used to execute program code on the terminal. Further, memory **330** may be used on terminal **300** to store such program code and further to locally store data in some cases. Memory **330** may be any tangible, non-transitory storage medium in one embodiment.

Further, terminal **300** may have a user interface/display **340**. The user interface/display may, in one embodiment, include separate input and output mechanisms, for example a keyboard, mouse, stylus, microphone, among others as the input mechanism, and a display and/or audio output as the output mechanism. In other embodiments the input and output mechanisms may be combined, for example on a touch screen.

The various elements of terminal **300** may communicate with each other, for example through a bus **350**. However, other options are possible.

The present disclosure will be further described in reference to specific databases used in a hospital setting. However, this example is provided for illustrative purposes only and is not intended to be limiting.

In a typical hospital setting, data is stored in four databases, namely Utilization Management / Clinical Criteria (UM/CC), Bed Board (BB), Dashboard and Analytics (DA) and Assessments (AS). Currently, all four of these systems, namely UM/CC, BB, DA and AS, are provided independently. This has the drawback that data from one module is not used to inform decisions related to another module, which leads to sub-optimal resource allocation and increased costs.

The present disclosure overcomes these drawbacks by providing a fully integrated database including each of UM/CC, BB, DA and AS. Each of these modules will now be described in detail.

In particular, a terminal may display a dialog based on data received from any of the UM/CC, BB, DA and AS modules, either individually or in combination. This allows a user to have an integrated view of the most relevant data for a particular task, regardless of whether this data is originally stored in one module or another.

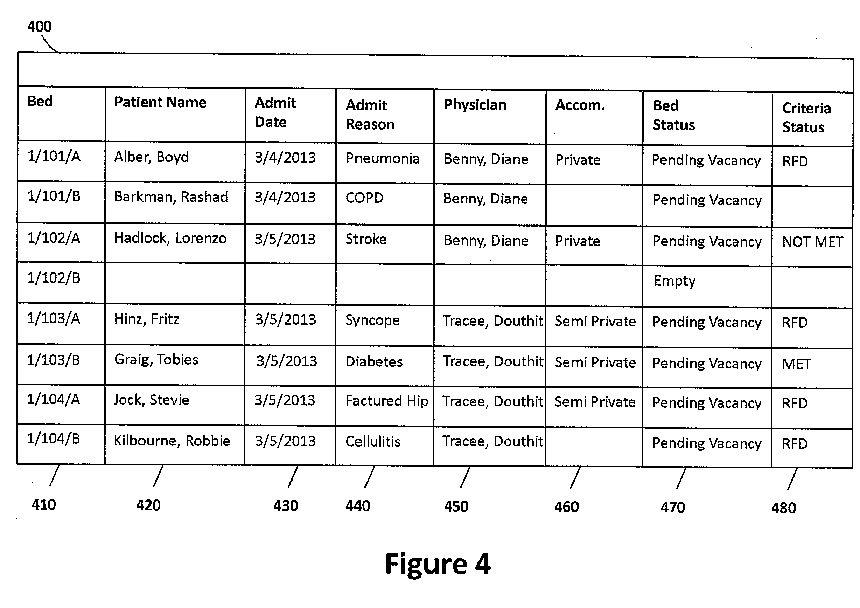

Thus, according to at least one embodiment, a terminal may be configured to request some data from the UM/CC module, and some data from the BB module, correlate the data, and present the data in an appropriate dialog box. An example of such a dialog box is shown with reference to **Figure 4**. Notably, in some embodiments, the terminal may also receive data and store it in the appropriate database or module.

In other cases, data may be changed at a terminal **300** and such data may be propagated to the appropriate database, for example using a network **105** from **Figure 1**.

In particular, **Figure 4** shows a dialog **400** displaying data from the BB module and the UM/CC module. In the example shown, columns **410**, **420**, **430**, **440**, **450**, **460** and **470** relate to the BB module. However, column **480** includes data taken from the UM/CC module.

Importantly, to present data as in **Figure 4**, in which some data relates to a first database or module and other data relates to a second database or module, the data is correlated, so that data from a first database may be presented along with appropriate data from a second database, as in **Figure 4**.

Thus, in **Figure 4**, the data from column **480**, "Criteria Status", is presented in a table such that we know the status of patient Boyd Alber is "RFD", even though the name of the patient is taken from the BB database and the status is taken from the UM/CC module.

According to one embodiment, this is accomplished by maintaining a special key value in every database. Thus, for the above example, BB data related to patient Boyd Alber is tagged with an identifier, and the UM/CC data related to Boyd Alber is tagged with the same identifier. When a terminal requests data from the BB module it receives the data along with the identifier for each piece of data. The terminal may then request data from the UM/CC module which corresponds to the received identifiers.

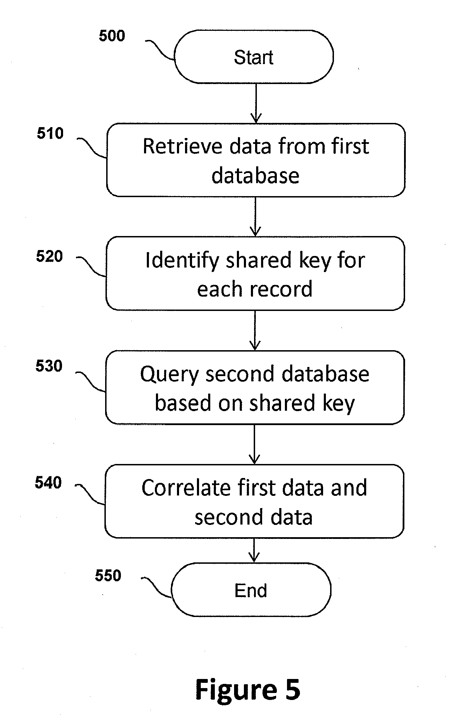

This process is illustrated with reference to **Figure 5**. The process starts at block **500** and proceeds to block **510** in which data from a first database is retrieved. This could be, for example, BB data with respect to patient Boyd Alber. Alternatively, this could be BB data for a number of patients, or another kind of data entirely.

Then, at block **520**, the retrieved data is inspected and a shared key is identified for every record. In one embodiment, the shared key may be marked with a special indicator. Alternatively, the terminal may have been preprogrammed to know which field of each record serves as a shared key. Thus, according to the above example, where the first data is BB data with respect to patient Boyd Alber, the terminal may be aware that the shared key for a patient record is stored in the "patient-ID" field.

At block **530**, the terminal queries a second database for records containing the shared key. Thus, according to the above example, a query is made to a UM/CC database for data records which have the value of "patient-ID" corresponding to patient Boyd Alber. In this manner, BB data for patient Boyd Alber is correlated with the correct UM/CC data. More generally, data from a first database is correlated with data from a second database.

Thus, at block **540**, the terminal establishes the correlation between data from the first database and data from the second database, and the process ends at block **550**.

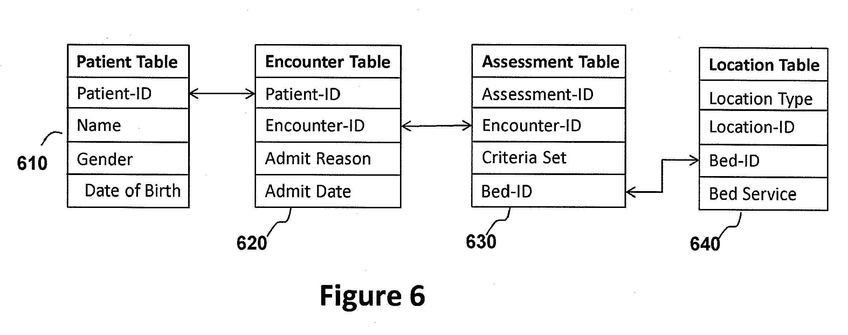

In some embodiments, data from a first database may be correlated with data from an arbitrary number of databases, as illustrated in **Figure 6**.

**Figure 6** illustrates 4 tables, namely a patient table **610**, an encounter table **620**, an assessment table **630** and a location table **640**. As will be appreciated by those skilled in the art, the tables of **Figure 6** are provided for illustrative purposes only, and are not limiting to the present disclosure.

The tables of **Figure 6** include patient objects, encounter objects, assessment objects, and location objects, respectively. For simplicity, no objects are depicted therein, and only the attributes of the tables are provided.

Patient table **610** describes patients with a patient-ID, a name, a gender and a date of birth.

Notably, the patient-ID attribute also appears in the encounter table **620**, as illustrated by an arrow. Thus the patient-ID attribute allows a correlation between a patient object and an encounter object.

Similarly, encounter table **620** includes an encounter-ID attribute, which is also present in assessment table **630**. Thus, the encounter-ID attribute allows a correlation between an encounter object and an assessment object. As per the patient-ID in tables **610** and **620**, a correlation between a patient object and an assessment object is also created.

Similarly, assessment table **630** includes a bed-ID which is shared with location table **640**. As will be appreciated the number of correlations between data is not limited, and the present disclosure is applicable to an arbitrary number of correlations.

### Utilization Management / Clinical Criteria (UM/CC)

Utilization Management (UM/CC) relates to clinical patient data and identifies whether a patient is receiving an appropriate level of care, and whether they are currently admitted at the correct level of care.

UM/CC data is collected daily, starting from the admission of a patient. According to at least one embodiment, collection of UM/CC data consists of performing a status assessment of a patient against a clinical criteria set. Clinical criteria sets allow a caregiver/hospital to determine whether the level of care afforded to a patient is appropriate, and are discussed in greater detail below.

Patient information is provided to the UM/CC module from the ADT system **202** from **Figure 2** above.

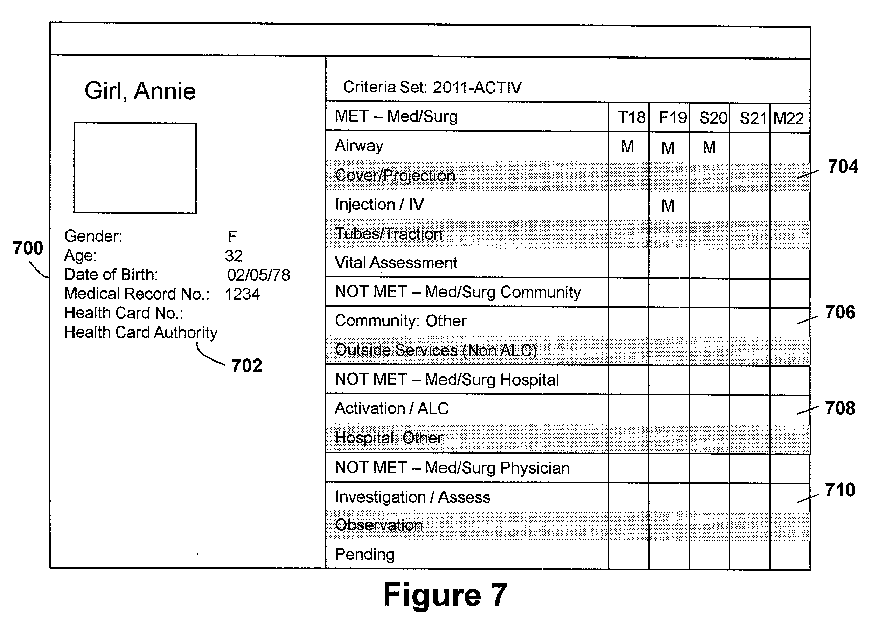

Reference is now made to **Figure 7**, which shows a user interface displayed by a terminal **104** and presenting UM/CC data. According to at least one embodiment, the UM/CC data displayed in **Figure 7** is received from the ADT system **202**.

The report **700** relates to patient Annie, which is identified in the patient identification section **702**.

Section **704** lists criteria for keeping a patient in their current level of care. In at least one embodiment, as long as one of these criteria is met each day, the patient is deemed to be receiving a proper level of care.

Sections **706, 708** and **710** list statuses when the patient is not meeting the appropriateness criteria for the level of care. Thus, if none of the criteria in section **704** are met, other criteria will help determine what barrier or delay is stalling the patient flow, thereby not allowing the plan of care to progress and/or preventing the patient from moving to a different level of care. Other levels of care may, for example, include Continuing Complex Care or in the community with services.

In at least one embodiment, the user interface of **Figure 7** allows the patient status to be edited, as is known in the art.

Notably, the user interface of **Figure 7** allows improvements in the discharge planning process, as it allows for an easy identification of the barriers to discharge. For example, using the user interface of **Figure 7**, and relying on their clinical expertise, nurses may determine whether the patient has met criteria indicating the patient is receiving the appropriate level of care. This also allows nurses, and the clinical team, to identify barriers, interruptions and delays to the patient's progress to discharge.

The UM/CC data may further be compiled to provide reports to management, allowing for trends to be analyzed, capacity to be predicted, and performance measured. In particular, the UM/CC data as described above allows the present system to keep track of patients who spend days in the hospital while being ready for discharge or for an alternate level of care, thereby identifying potential improvements.

According to at least some embodiments of the present disclosure, the UM/CC data comprises clinical criteria sets. Clinical criteria sets lay a common framework for multi-disciplinary dialogue on clinical status, providing confidence and consistency in patient assessment. The criteria are researched from international best practices and based on a) intensity of service requirements of the patient; and b) severity of illness of the patient. The two part assessment identifies firstly the appropriateness of the patient for their current level of care (setting) and secondly their readiness for a safe discharge/transition to another level of care. In at least some embodiments, built-in criteria sets designed for specific types of patients are provided. For example, these built-in criteria sets may include, but are not limited to, criteria sets for pre-admittance patients, medical-surgical patients, ICU, mental health patients, pediatrics, newborns, post partum, complex continuing care, and rehabilitation.

Thus, for every type of patient, there is provided a clinical criteria set against which a health-care provider can determine whether an appropriate level of care is provided, determine readiness for discharge, and identify barriers and delays. The clinical criteria set are built-in to the present system and the barriers and delays (reasons and details) may be customized by hospital staff.

According to at least one embodiment, a clinical criteria set includes a first subset of criteria directed to determining whether an appropriate level of care is provided. Such criteria can include, for example, whether a patient requires an IV. In at least one embodiment, if at least one criteria of this first subset of criteria is met, the patient is determined to receive the appropriate level of care.

According to at least one embodiment, a clinical criteria set includes a second subset of criteria directed to identifying barriers or delays. Specifically, if none of the criteria in the first subset are met, this may be because a patient is ready for discharge, and/or it may be because some barriers are preventing the proper progression of the patient's plan of care. For example, the results of a test performed on the patient may not have been returned from a hospital laboratory on time. Therefore, a criteria of the second subset of criteria could be whether a laboratory result is missing.

In at least one embodiment, criteria of the first subset are selected when they are met (e.g., the patient meets the criteria of needing an IV).

In at least one embodiment, a clinical criteria set further includes a readiness for discharge (RFD) test. Thus, when none of the criteria of the first subset are met, and none of the criteria of the second subset are not met, this suggests that a patient is ready for discharge. The RFD test may include questions about the patient's condition and symptoms. If at least one of the criteria of the second subset are not met then the patient is considered not ready for discharge (NRFD).

Accordingly, the present disclosure provides for means of tracking whether a patient has appropriate level of care, identifying barriers to treatment, and determining whether a patient is ready for discharge. This data is maintained by the UM/CC module, and shared with other modules, such as the Bed Board (BB) module described in greater detail below.

In particular, whereas the BB module tracks status of resources (i.e. beds) and the allocation of resources to patients, the UM/CC data, and in particular the status of a patient with respect to its criteria set and its readiness to discharge, allows the BB module to predict future resource availability. Thus, in at least one embodiment, the BB module is integrated with the UM/CC module and publishes, in dialogs such as that shown in **Figure 4**, a "Criteria Status", whereas the Criteria Status indicates that an appropriate level of care is being provided (i.e., "MET"), that a delay or barrier has been identified, (i.e., "NOT MET"), and that the patient is ready for discharge (i.e., "RFD") or not ready for discharge (i.e. "NRFD").

This information when provided to the BB module improves planning capacity, thereby including the quality of care in the decision making and reducing waste and streamlining operations.

### Bed Boards (BB)

The BB module provides concurrent clinical and operational status of patients per location and resources, helps manage beds, predicts bed demand and capacity, displays readiness for discharge, and ensures strategic, operational and clinical visibility at all points in the patient journey.

Amongst other things, the BB module keeps track of patients without beds, empty beds, occupied beds, bed requests, including bed requests with special conditions, such as a private room or specialized equipment.

The BB module further keeps track of each patient assigned to a bed and which bed each patient is assigned to.

The UM/CC data may also be used to improve decisions in the Bed Board (BB) module. An example of the integration of the BB module and the UM/CC module is shown with reference to **Figure 4**. **Figure 4** shows a report displayed on a terminal 104, which lists all admitted patients, their bed number, and importantly, their UM/CC status.

In at least one embodiment, the UM/CC status may take one of the following values: ready for discharge (RFD), not ready for discharge (NRFD), ready for transfer (RFT), MET and NOT-MET. A MET status signifies that the patient is receiving the appropriate level of care. A NOT-MET status signifies that a barrier, delay or interruption to the care has been identified. An RFD status indicates that the patient is ready for discharge from the current care setting, and the NRFD status indicates that a patient still has unmet needs before discharge or transition to a new level of care.

By identifying all patients which are RFD or RFT, the UM/CC module, when integrated with the BB module, provides users with an estimate of which beds will be freed on a given day. This allows for improved planning and resource allocation.

Therefore, the BB module, when integrated with the UM/CC module, allows for:
- forecasting and managing demand and capacity, by measuring and predicting multiple sources of demand, including external, outpatient, surgical and internal;
- addressing bottlenecks in patient flow;
- differentiating between specific types of capacity and resource availability;
- seamlessly help move the plan of care for each patient forward;
- reducing delays and improper bed placement;
- gaining comprehensive big-picture views of the unit, hospital or region.

### Dashboard and Analytics (DA) functionality is available for all modules

A further module of the present disclosure is Dashboards and Analytics (DA). DA is a clinical business intelligence tool ensuring timely data is used to support informed decisions. In at least one embodiment, DA comprises a configurable graphic dashboards and reports for providing data analysis. In at least one embodiment, DA provides performance indicators.

In at least some embodiment, the DA module performs one or more of the following tasks:
- breaks down data by service, program, and occupancy rate;
- predicts discharge data and compares predictions to actual data;
- provides admission and discharge data by day of the week;
- computes the average length of stay;
- computes 7-day and 30-day readmission rates;
- keeps track of discharge delays and bed turnaround times.

According to at least one embodiment, the DA module allows each user of the system to customize a dashboard or report, for displaying selected data and performing selected analysis of this data.

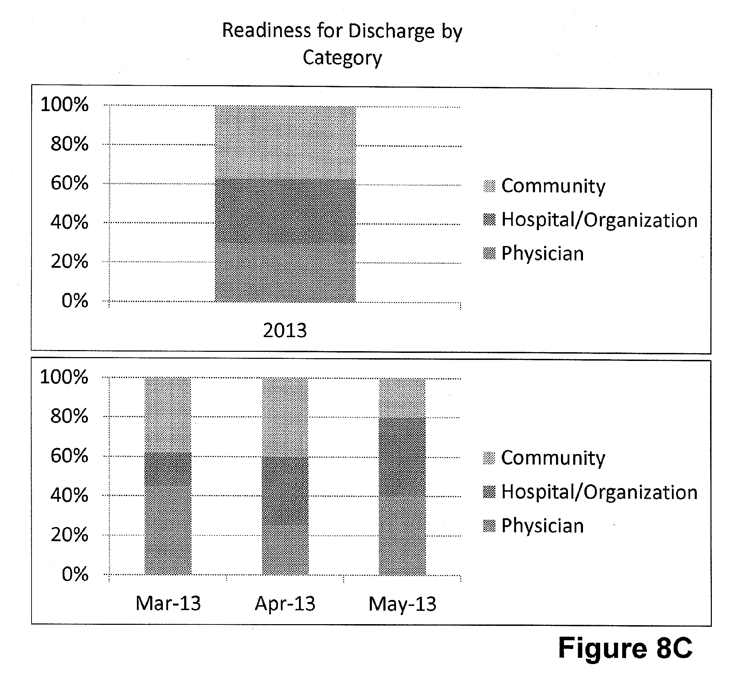

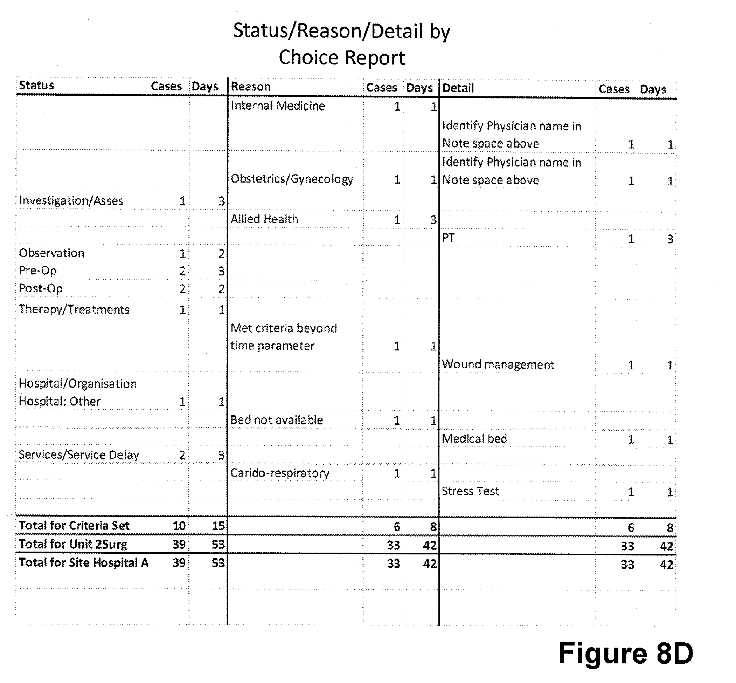

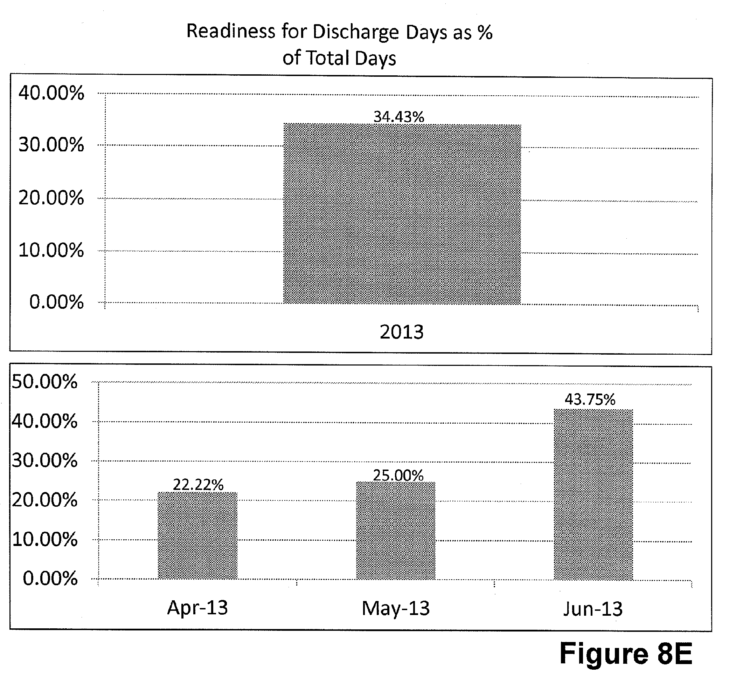

Examples of such Dashboards and Reports are included herein as :

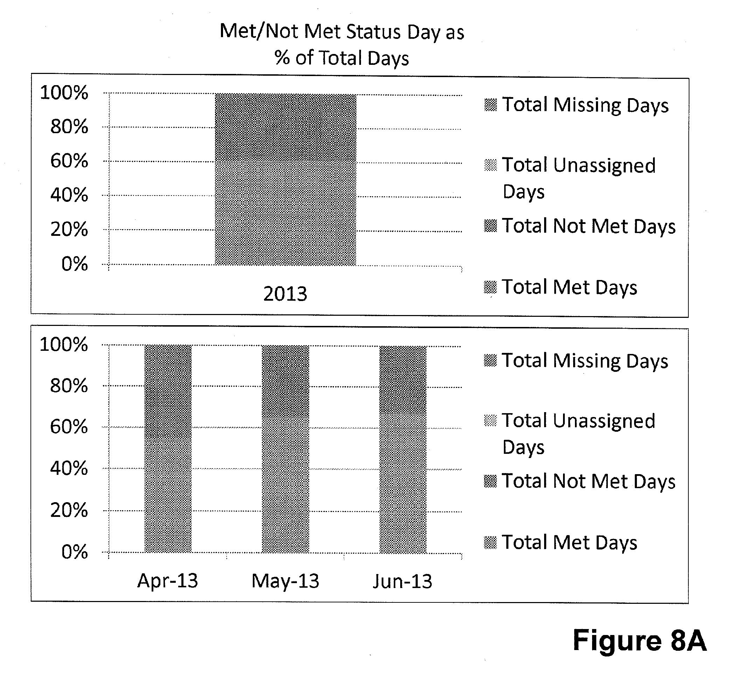

Figure 8A - Met vs Not-Met
   - Graphical display by year and by month of % of patient days of stay meeting criteria (appropriate days of staying at the current level of care) and days not meeting criteria (days of stay not at the appropriate level of care)

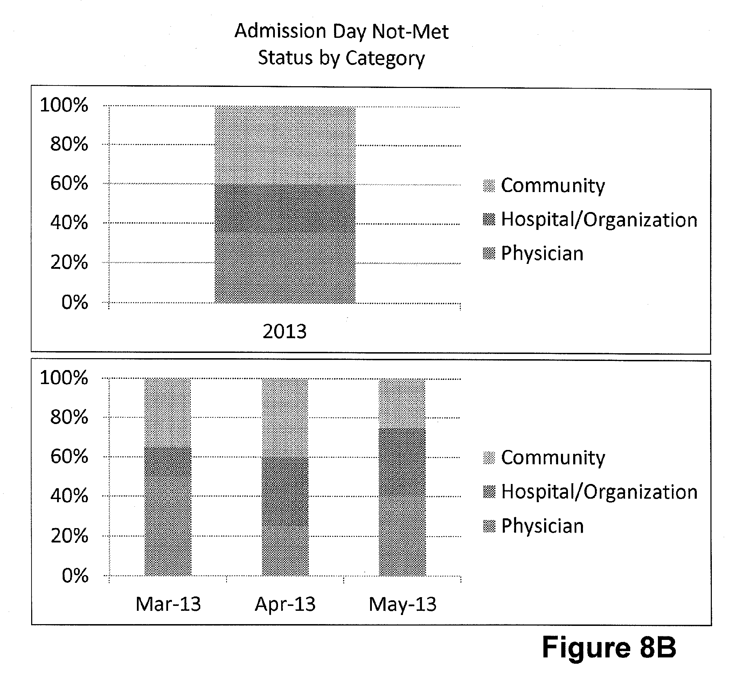

Figure 8B - Admission Day Not-Met Graph
   - Graphical display by year and by month of % of admission days not meeting criteria (days of stay not at the appropriate level of care) divided into delay reasons according physician, hospital or community related
Figure 8C - RFD By Category
   - Graphical display by year and by month of % of patient days of stay not meeting criteria and ready for discharge (days of stay not at the appropriate level of care) divided into delay reasons according physician, hospital or community related
Figure 8D - Status Reason Details
   - Summary display by count of days of stay by reasons for delay
Figure 8E - RFD Total Days
   - Graphical display by year and by month of % of patient days of stay not meeting criteria and ready for discharge (days of stay not at the appropriate level of care)

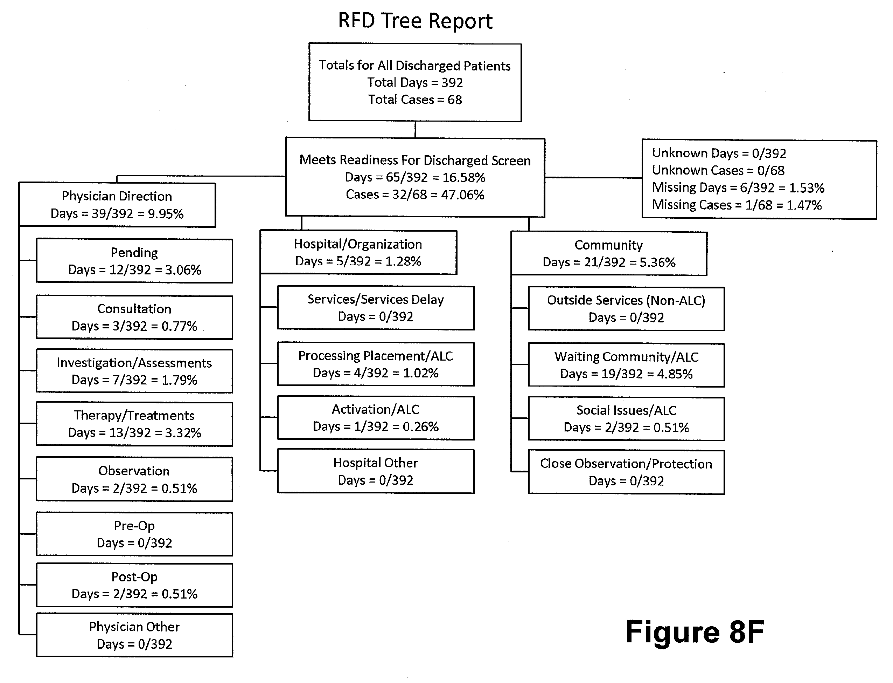

Figure 8F - RFD Tree Report
   - A Summary unique "Tree" format display of % of patient days of stay not meeting criteria and ready for discharge (days of stay not at the appropriate level of care) divided into delay reasons according physician, hospital or community related and broken into their sub statuses

### Assessments

A further module included in the present system and integrated with the other modules is the Assessments module (AS). The AS module is a tool for the generation of flexible and customizable electronic forms for entering patient clinical data. The flexibility and customizability of AS allows it to fit the hospital and the patients' needs and improve patient flow in such areas as discharge planning, clinical workflow management, and performance measurement. This information also is displayed on the BedBoard and enhances the bed management and care management processes for improving patient flow.

In particular, the AS module enables integration of key assessments and forms into the care delivery process, thereby allowing smoother transitions between levels of care and improved patient safety.

The AS module further provides for ad hoc data collection and reporting needs. In particular, in at least one embodiment, the AS module comprises a form generator allowing a user of the system to generate a form which is specific to an individual patient's needs. In at least one embodiment, the form generator allows for the incorporation of data from multiple data sources within the system, including integrated modules UM/CC, and BB and DA, as well as from HIS 102.

In at least one embodiment, the AS module further includes a graphical engine, for representing data in the form of graphical charts or graphs.

In at least one embodiment, the AS module further allows for a user to access data via the Internet using a web interface, and to export data in a plurality of common formats, such as extensible markup language (XML), hypertext markup language (HTML), portable document format (PDF), Microsoft® Word and Microsoft® Excel, among others.

In at least some embodiments, the AS module complies with standardized Alternate Level of Care (ALC) for identification of patients designated as requiring an alternate care level as they no longer require the current level of care.

The AS module can further be integrated with other modules such as the UM/CC module described above. In particular, UM/CC data may be bound to AS forms, thereby reducing redundant entries.

The above therefore provides a system and method for integrating multiple databases in a system. While the above describes some elements by way of example, the present disclosure is not limited to any specific example but rather by the concepts described therein.

## Claims

1. A method for integrating multiple databases, comprising:
requesting, from a terminal, data from at least one server, the at least one server maintaining a first database and a second database;
receiving at the terminal the requested data;
correlating data at the terminal from the first database with data from the second database; and
displaying data at the terminal from the first database with correlated data from the second database.

2. The method of claim 1, wherein first data is requested from the first database and second data is requested from the second database.

3. The method of claim 2, further comprising, displaying the first data and the second data in a table.

4. The method of any one of claims 1 to 3, wherein each entry of the first database comprises a first identifier, and each entry of the second database comprises a second identifier.

5. The method of claim 4, wherein the first data is correlated with the second data by matching the first identifier and the second identifier.

6. The method of any one of claims 1 to 5, wherein the first database is a Clinical Criteria (CC) data and the second database is a Bed Board (BB) database.

7. The method of claim 6, further comprising a Hospital Information System (HIS) in communication with the at least one server.

8. The method of any one of claim 1 to 7, wherein said requesting and said receiving use the Health Level 7 (HL7) protocol.

9. The method of any one of claims 1 to 8, further comprising the steps of:
receiving user input;
editing one of the first database and the second database based on the user input.

10. A system for integrating multiple databases, comprising:
at least one server, the at least one server maintaining a first database and a second database; and
a plurality of terminals, each of the plurality of terminals comprising a processor configured to perform the method of any one of claims 1 to 9.

11. The system of claim 10 wherein the plurality of terminals are one of a laptop computer, a desktop computer, a mobile device, and a specialized computing appliance.
